# EUROPÄISCHE PATENTSCHRIFT

(11) **EP 4 088 765 B1**
(45) Veröffentlichungstag und Bekanntmachung des Hinweises auf die Patenterteilung: **20.12.2023**
(21) Anmeldenummer: 21173069.2
(22) Anmeldetag: 10.05.2021
(51) Int. Cl.: A61M 5/315, A61M 5/31

(54) **EINWEG-INJEKTIONSVORRICHTUNG**
DISPOSABLE INJECTION DEVICE
DISPOSITIF D'INJECTION JETABLE

(43) Veröffentlichungstag der Anmeldung: 16.11.2022
(73) Patentinhaber: Telamedica GmbH, 8004 Zürich (CH)
(72) Erfinder: SNOZZI, Philippe, 8004 Zürich (CH)
(74) Vertreter: Schirbach, Marcel

(56) Entgegenhaltungen:
- GB-A- 795 202
- US-A- 2 842 128
- US-A- 4 217 896
- US-A- 4 351 334
- US-A- 4 687 472
- US-B1- 10 980 945

## Beschreibung

### Technisches Gebiet

Die vorliegende Erfindung fällt in den technischen Bereich der medizintechnischen Vorrichtungen; sie betrifft eine Einweg-Injektionsvorrichtung gemäss dem Oberbegriff des Anspruchs 1.

### Stand der Technik, technologischer Hintergrund der Erfindung

Spitzen sind bekannte, medizinische Instrumente, die zur Verabreichung von Medikamenten, Nähr- oder Infusionslösungen, zur Entnahme von Körperflüssigkeiten oder Körpergewebe, sowie zum Spülen, beispielsweise von Wunden, Verwendung finden. Neben den nach entsprechender Sterilbehandlung wiederverwendbaren Spritzen sind auch Einweg-Injektionsvorrichtungen, bzw. Einwegspritzen weit verbreitet; solche Einwegspritzen sind in der Regel einzeln und steril verpackt. Üblicherweise bestehen Einwegspritzen aus Kunststoff und umfassen zumeist mindestens zwei Teile, nämlich einen Zylinder und einen Kolben; es sind auch Einwegspritzen erhältlich, deren Kolben einen dichtenden Gummistopfen besitzen oder bereits mit einer eingeklebten Kanüle vollständig ausgebildet sind; bekannte Einwegspritzen können dann auch die zu verabreichende Injektionslösung bereits beinhalten, wie beispielsweise Hyaluronsäurespritzen für den Kosmetikbereich, oder Insulinspritzen für die Diabetesbehandlung.

Einwegspritzen unterstehen -wie grundsätzlich alle medizinischen Instrumente- aufwendigen Zulassungsverfahren, die regulär nationaler, regionaler oder internationaler Gesetzgebung unterstehen, wobei jedwede wünschenswerte Änderung an solchen Einwegspritzen nicht nur hinsichtlich etablierter Produktionslinien aufwendig ist, sondern darüber hinaus vorgängig immens zeit- und kostenintensive Neuzulassungsverfahren mit sich bringen. Vor diesem Hintergrund ist es deshalb bei weitem einfacher, ergänzende Vorrichtungen an Einwegspritzen, beispielsweise für die Aspiration, als separates Teil auszufertigen, welches unmittelbar im Einsatzfall an der Einwegspritze angeordnet und verwendet wird.

Nicht nur aus der Druckschrift EP 2 896 421 A1 ("Barbour") ist bekannt, dass - neben dem Gebot der Medizin, jeden Patienten mit kleinstem Risiko und grösstmöglicher Sicherheit zu behandeln- grundsätzlich auch viel Wert auf den Komfort eines Patienten gelegt wird, der beispielsweise vorliegend eine Injektion erhalten soll. Auf der anderen Seite erscheint der Komfort einer behandelnden Person, die die Spritze verwendet, diesbezüglich bis anhin nicht so bedeutungsvoll zu sein; allerdings kann davon ausgegangen werden, dass sich der Komfort eines Patienten auch mit dem gesteigerten Komfort der die Spritze setzenden Person erhöhen lässt. Dies kann besonders relevant sein, wenn zahlreiche Injektionen mittels kleiner zu injizierender Volumina im Kosmetikbereich vorgenommen werden sollen. Vor diesem Hintergrund erfahren besonders ergonomisch angepasste bzw. ausgestaltete Einwegspritzen mehr und mehr an Bedeutung. In diesem Zusammenhang wird für die behandelnde Person ein Daumenring oder ein Daumengriff am Spritzenkolben für eine einhändige Aspiration als besonders vorteilhaft empfunden; jedoch ist der Anteil Einwegspritzen mit Daumenring mit Blick auf die bekannte Marktsituation immer noch bedeutend kleiner als der Anteil Einwegspritzen ohne Daumenring, weshalb in der vorliegenden EP 2 896 421 A1 ein auf Einwegspritzen ohne Daumenring nachträglich montierbarer Daumenring offenbart wird, welcher kurz vor dem Einsatz an einer solchen Einwegspritze ohne Daumenring ergänzt wird. Der Umstand, dass solche Daumenringe als ergänzendes Extra überhaupt in Erwägung gezogen werden, ist auch vorliegend im Wesentlichen den oben bereits erwähnten, immens aufwendigen Zulassungsverfahren geschuldet. Somit kann weiterhin auf zulassungskonforme Einwegspritzen ohne Daumenring abgestellt werden, die dann mittels separatem Daumenring ergänzt werden können. Auch die Druckschrift US 2015/0238698 A1 ("Allergan") offenbart einen separaten Daumenring, der an einem Kolben einer Einwegspritze in Einsatzfall montierbar ist.

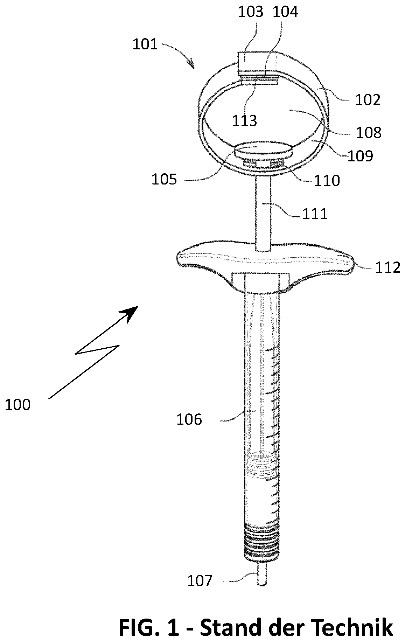

Aus der Druckschrift US 10,980,945 B1 ("Liftie") ist ferner ein separates, ebenfalls nachträglich an eine Einwegspritze 100 montierbares Stretchband 102 bekannt, wie aus der nachfolgenden Fig. 1 zum Stand der Technik hervorgeht: Das Stretchband 105 weisst hierfür einen Schlitz 110 auf, durch den ein Kolbenkopf 105 der Einwegspritze 100 hindurchführbar ist, ganz ähnlich einer Hemdkopfanordnung, bei welcher ein Hemdknopf durch ein Knopfloch zum Verschliessen eines Hemdes führbar ist. Das Stretchband 102 umfasst einen ersten und einen zweiten Bandverschluss 104, 113, die miteinander verbindend als ein Klettverschluss ausgebildet sind. Mittels der hier vorgestellten Bandanordnung 101 kann jede einfache Einwegspritze 100 mit einer als ein Daumenring fungierende Aufnahmevorrichtung ausgestattet werden, um für eine behandelnde Person die einhändige Aspiration ermöglichen zu können.
Das Dokument US 2,842,128 zeigt eine zur Mehrfachverwendung geeignete Spritze, an deren Kolbenkopf ein Daumenring in Form einer Klammer montierbar ist, wobei die mit Greiflippen versehenen Arme der Klammer mittels einer Schraubverbindung an den Kolbenkopf angedrückt oder von ihm gelöst werden können.

Dem gesamten oben gewürdigten Stand der Technik ist grundsätzlich gemeinsam, auf Wunsch, herkömmliche Injektionsvorrichtungen nachträglich mit einer separaten Vorrichtung ergänzend derart auszustatten, dass eine einfache einhändige Aspiration überhaupt ermöglicht wird, wobei ohne näher auf einen jeweiligen, nationalen Zulassungsstatus solcher zusätzlichen Vorrichtungen einzugehen, zumindest von jeweils vollständig zugelassenen Einwegspritzen ausgegangen werden kann. Ein grundsätzlich hervorzuhebendes Kriterium von Einwegspritzen ist, dass diese typischerweise für einen weiteren Gebrauch nicht vorgesehen sind; aus diesem Grund können Einwegspritzen zur Verhinderung einer mehrfachen Verwendung beispielsweise Mittel aufweisen, die solche Mehrfachverwendungen verunmöglichen. Im Gegensatz zu solchen Einwegspritzen, die entsprechende Zulassungsbedingungen hinsichtlich untersagter bzw. unterbundener Mehrfachverwendungsmöglichkeiten erfüllen, werden die aus dem Stand der Technik bekannten, separat montierbaren Aspirationsvorrichtungen solchen Bedingungen keinesfalls gerecht; ganz im Gegenteil, sämtliche konventionellen, separat montierbaren Aspirationsvorrichtungen werden sogar als montierbar und demontierbar beschrieben, womit die Mehrfachverwendung dieser nachträglich anordenbaren Aspirationsvorrichtung als eingeräumt erscheinen kann, was darüber hinaus mit Blick auf medizinische Zulassungsbedingungen hinterfragenswert erscheinen kann.

### Darstellung der Erfindung

Es ist deshalb Aufgabe der vorliegenden Erfindung, eine Einweg-Injektionsvorrichtung dahingehend weiterzuentwickeln, dass neben der Bereitstellung einer nachträglichen Ergänzung für eine einhändige Aspiration, darüber hinaus auch wesentliche Zulassungsbedingungen für die Injektionsvorrichtung hinsichtlich einer Mehrfachverwendung, auch von diesen nachträglichen Ergänzungen für eine einhändige Aspiration, erfüllbar sind.

Die der Erfindung für die Einweg-Injektionsvorrichtung zugrundeliegende Aufgabe wird gelöst durch die Merkmale des Anspruchs 1; die diesen Erfindungsgedanken weiterbildenden Merkmale sind jeweils Gegenstand der Unteransprüche 2 bis 4.

Der Kern der vorliegenden Erfindung ist darin zu sehen, dass eine Einweg-Injektionsvorrichtung, die einer einfachen einhändigen Aspiration nicht unmittelbar zugänglich ist, mittels einer nachträglich an dieser Einweg-Injektionsvorrichtung anordenbaren, separaten Aufnahmevorrichtung ausstattbar sind, wobei diese Aufnahmevorrichtung nach einer ersten und damit einmaligen Montage, nicht mehr zerstörungsfrei von dieser Einweg-Injektionsvorrichtung trennbar ist. Mittels dieser erfindungsgemässen Einweg-Injektionsvorrichtung kann somit erstmalig sichergestellt werden, dass nach Gebrauch diese Einweg-Injektionsvorrichtung mit daran angeordneter Aufnahmevorrichtung als Ganzes, einer Mehrfachverwendung keinesfalls mehr zugänglich ist.

Die erfindungsgemässe Einweg-Injektionsvorrichtung mit einem Zylinderabschnitt und einer darin angeordneten Kolbenanordnung, welche mit einem ersten Ende den Zylinderabschnitt verschliesst und an einem zweiten Ende einen Daumenstempel zwecks Bedienung dieser Kolbenanordnung im Zylinderabschnitt aufweist, umfasst eine an diesem Daumenstempel separate montierte Aufnahmevorrichtung, die derart konfiguriert ist, einen Daumen eines Benutzers zwecks einhändigen Ansaugens während des Betriebs der Einweg-Injektionsvorrichtung mit dieser Kolbenanordnung wirkverbunden anzuordnen, wobei die separate Aufnahmevorrichtung mittels einer eine dauerhafte bzw. nicht wieder lösbare Verbindung ausbildende Rastelementanordnung an dem Daumenstempel montierbar ist.

Bei einer vorteilhaften Ausführungsform der erfindungsgemässen Einweg-Injektionsvorrichtung ist vorgesehen, dass die Aufnahmevorrichtung einen Daumenring und eine dem Daumenstempel zugewandte Auflagefläche aufweist, die an mindestens zwei einander gegenüberliegenden Auflageenden Einfasswinkel umfasst, wobei an diesen Einfasswinkeln die Rastelementanordnung den Daumenstempel einfassend ausgebildet ist, womit die Aufnahmevorrichtung an dem Daumenstempel nicht wieder lösbar ausgestaltet ist.

Bei einer weiteren Ausführungsform ist mit Vorteil vorgesehen, dass die Einfasswinkel jeweils mindestens eine Öffnung aufweisen, durch die die als eine Anzahl Rastelemente ausgebildete Rastelementanordnung bedienbar angeordnet ist.

Mit Vorteil ist der Daumenstempel bei einer weiteren, erfindungsgemässen Ausführungsform zwischen diesen Rastelementen und der Auflagefläche der Aufnahmevorrichtung unlösbar fixierbar.

Besonders vorteilhaft ist eine weitere Ausführungsform der Erfindung derart ausgestaltet, dass die Aufnahmevorrichtung mindestens eine Sollbruchstelle aufweist, sodass jede Absicht, die Aufnahmevorrichtung von der Einweg-Injektionsvorrichtung zwecks einer mutmasslichen Wiederverwendung damit geahndet wird, dass diese Aufnahmevorrichtung unwiderruflich zerstört wird. Eine solche Sollbruchstelle kann beispielsweise am Daumenring vorgesehen sein, welche von der ihn mit dem Kolbenstempel verbindenden Auflagefläche bei unsachgemässer Krafteinwirkung - gleichwohl in welche Kraftrichtung auch immer- abtrennt. Ferner kann eine gleichwirkende Sollbruchstelle auch oder zudem an einem oder beiden Einfasswinkeln vorgesehen werden.

Bei der erfindungsgemässen Einweg-Injektionsvorrichtung ist eine Anzahl Führungselemente vorgesehen, die den Daumenstempel neben den Rastelementen zusätzlich seitlich einfassen, um zusätzlich ein seitliches Herausschieben des Daumenstempels aus der Aufnahmevorrichtung zu verunmöglichen.

Ohne den vorliegenden grundsätzlichen Erfindungsgedanken zu verlassen ist es selbstverständlich denkbar, dass der Daumenstempel jedwede geometrische Form aufweisen kann und die Aufnahmevorrichtung ohne jeden weiteren erfinderischen Beitrag derart auszugestalten ist, dass diese Aufnahmevorrichtung möglichst einfach und rasch, aber in jedem Fall unlösbar mit diesem Daumenstempel verbindbar ist. Erst mittels des vorliegenden erfinderischen Beitrags, der vorliegend über den bekannten Stand der Technik hinausgeht, ist die Erfüllung wesentlicher Zulassungsbedingungen auch für die Ergänzung für eine einhändige Aspiration an der Einweg-Injektionsvorrichtung gegeben.

### Kurze Beschreibung der Zeichnungen

Im Folgenden wird die Erfindung anhand von Figuren beispielhaft erläutert. Gleiche Gegenstände sind in den Figuren grundsätzlich mit gleichen Bezugszeichen versehen. An dieser Stelle wird darauf hingewiesen, dass die Figuren keinerlei einschränkende Wirkung auf den vorliegenden Erfindungsgegenstand als solches haben, sondern lediglich exemplarische Ausführungsformen des Erfindungsgedankens darstellen.

### Es zeigen rein schematisch die

Fig. 1 eine Einweg-Injektionsvorrichtung gemäss oben hiermit gewürdigtem Stand der Technik, anhand derer grundsätzliche Merkmale offenbart bzw. definiert werden;
Fig. 2 eine erfindungsgemässe Einweg-Injektionsvorrichtung in einer Längsschnittdarstellung;
Fig. 3 eine seitliche Explosionsdarstellung einer Aufnahmevorrichtung für die erfindungsgemässe Einweg-Injektionsvorrichtung nach Fig. 2;
Fig. 4 eine perspektivische Explosionsdarstellung der Aufnahmevorrichtung für die erfindungsgemässe Einweg-Injektionsvorrichtung nach Fig. 3; und die
Fig. 5 eine weitere perspektivische Darstellung der Aufnahmevorrichtung für die erfindungsgemässe Einweg-Injektionsvorrichtung nach Fig. 4.

### Wege zur Ausführung der Erfindung

Fig. 1 zeigt eine Einwegspritze 100, wie sie aus dem Stand der Technik bereits bekannt und oben beschrieben ist; diese Fig. 1 dient vorliegend lediglich einer präzisierenden Definition des zugrundeliegenden, konventionellen Standes der Technik.

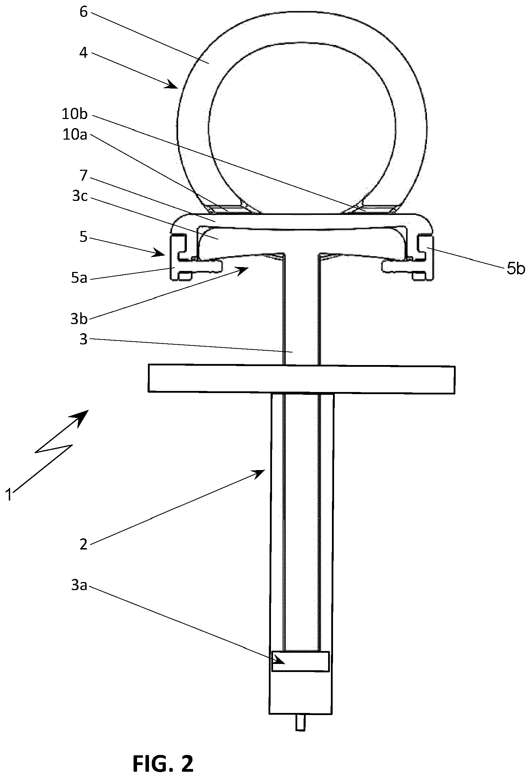

In Fig. 2 ist eine erfindungsgemässe Einweg-Injektionsvorrichtung 1 in einer Längsschnittdarstellung gezeigt. Diese Einweg-Injektionsvorrichtung 1 umfasst einen Zylinderabschnitt 2 und eine darin angeordnete Kolbenanordnung 3, welche mit einem ersten Ende 3a den Zylinderabschnitt 2 verschliesst und welche an einem zweiten Ende 3b einen Daumenstempel 3c zwecks Bedienung dieser Kolbenanordnung 3 im Zylinderabschnitt 2 aufweist. An diesem Daumenstempel 3c ist eine separate Aufnahmevorrichtung 4 montiert, die derart konfiguriert ist, dass ein -hier nicht näher gezeigter- Daumen eines Benutzers zwecks einhändigen Ansaugens während des Betriebs der Einweg-Injektionsvorrichtung 1 mit dieser Kolbenanordnung 3 wirkverbunden ist. Dabei ist diese Aufnahmevorrichtung 4 mittels einer dauerhaften bzw. nicht wieder zerstörungsfrei lösbaren Verbindung in Form einer Rastelementanordnung 5 an dem Daumenstempel 3c montiert. Wesentlich dabei ist, dass herkömmliche Einweg-Injektionsvorrichtungen 1 zum Zweck einer einfachen einhändigen Aspiration nachträglich mit dieser separaten Aufnahmevorrichtung 4 erweitert, ergänzt bzw. ausgestattet werden können, wobei diese Aufnahmevorrichtung 4 nach einer ersten und damit einmaligen Montage nicht mehr zerstörungsfrei von dieser Einweg-Injektionsvorrichtung 1 trennbar ist. Hierfür ist eine erste Sollbruchstelle 10a und/oder eine zweite Sollbruchstelle 10b an der Aufnahmevorrichtung 4 vorgesehen, die ein zerstörungsfreies Demontieren der Aufnahmevorrichtung 4 von der Einweg-Injektionsvorrichtung 1 unmöglich machen bzw. macht. Mittels dieser erfindungsgemässen Einweg-Injektionsvorrichtung 1 kann somit sichergestellt werden, dass nach einmaligem Gebrauch diese Einweg-Injektionsvorrichtung 1 mit daran angeordneter Aufnahmevorrichtung 4 als Ganzes, einer Mehrfachverwendung keinesfalls mehr zugänglich ist.

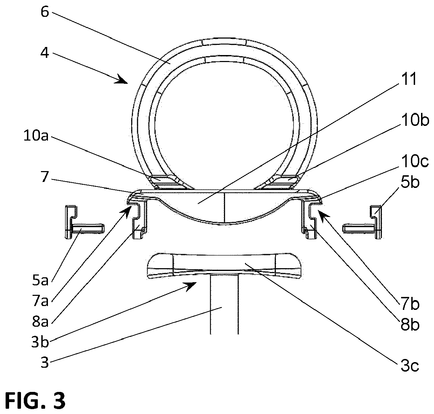

Bei der erfindungsgemässen Einweg-Injektionsvorrichtung 1 kann ferner vorgesehen sein, dass die Aufnahmevorrichtung 4 einen Daumenring 6 und eine dem Daumenstempel 3c zugewandte Auflagefläche 7 aufweist, die an mindestens zwei einander gegenüberliegenden Auflageenden 7a, 7b Einfasswinkel 8a, 8b aufweist, siehe hierzu auch die seitliche Explosionsdarstellung in Fig. 3. Vorliegend ist gezeigt, dass die Rastelementanordnung 5 mindestens ein erstes und/oder ein zweites Rastelement 5a, 5b umfassen kann, welche mittels der Einfasswinkeln 8a, 8b mit dem Daumenstempel 3c derart in Eingriff gelangen, dass dieser Daumenstempel 3c -quasi in einer Art Sandwich-Struktur- zwischen der Auflagefläche 7 und den Rastelementen 5a, 5b nach Montage der Aufnahmevorrichtung 4 fixiert ist, siehe Fig. 2.

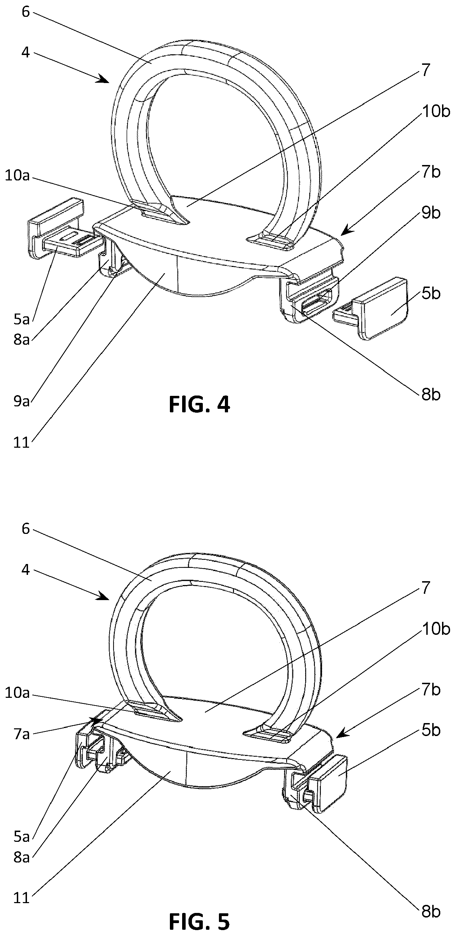

Fig. 4 zeigt eine perspektivische Explosionsdarstellung der Aufnahmevorrichtung 4 der erfindungsgemässen Einweg-Injektionsvorrichtung 1 nach Fig. 3, wobei für eine einfache Zugänglichkeit die Einweg-Injektionsvorrichtung 1 hier nicht explizit gezeigt ist. In dieser Darstellung sind Öffnungen 9a, 9b an den Auflageenden 7a, 7b sichtbar, in welchen Öffnungen 9a, 9b die Rastelemente 5a, 5b angeordnet sind, siehe hierzu auch die perspektivische Seitenansicht in Fig. 5, in welcher erkennbar ist, wie die Rastelemente 5a, 5b zumindest abschnittsweise in diesen Öffnungen 9a, 9b bereits eingeführt sind, so dass mittels dieser Anordnung eine unmittelbare Montage an einem Daumenstempel 3c vollziehbar ist.

Die Aufnahmevorrichtung 4 weist gegebenenfalls zudem eine Anzahl Führungselemente 11 auf, die den Daumenstempel 3c neben den Einfasswinkeln 7a, 7b zusätzlich einfassen können, um ein seitliches Herausschieben des Daumenstempels 3c aus der montierten Aufnahmevorrichtung 4 unterbinden zu können.

### Bezugszeichenliste

- 1: Einweg-Injektionsvorrichtung
- 2: Zylinderabschnitt
- 3: Kolbenanordnung
- 3a: erstes Ende der Kolbenanordnung 3
- 3b: zweites Ende der Kolbenanordnung 3
- 3c: Daumenstempel
- 4: Aufnahmevorrichtung
- 5: Rastelementanordnung
- 5a, 5b: Rastelement
- 6: Daumenring
- 7: Auflagefläche an Aufnahmevorrichtung
- 7a, 7b: Auflageende
- 8a, 8b: Einfasswinkel an Auflageende 7a, 7b
- 9a, 9b: Öffnung an Einfasswinkel 8a, 8b
- 10a, 10b, 10c: Sollbruchstelle
- 11: Führungselement

- 100: Einwegspritze
- 101: Bandanordnung
- 102: Stretchband
- 103: Bandaussenfläche
- 104: Bandverschluss
- 105: Kolbenkopf
- 106: Spritzenzylinder
- 107: Spitze
- 108: Daumenöffnung
- 109: Bandinnenfläche
- 110: Schlitz in Band 102
- 111: Kolben
- 112: Flansch an 106
- 113: Bandverschluss

## Patentansprüche

1. Einweg-Injektionsvorrichtung (1) mit einem Zylinderabschnitt (2) und einer darin angeordneten Kolbenanordnung (3), welche mit einem ersten Ende (3a) den Zylinderabschnitt (2) verschliesst und an einem zweiten Ende (3b) einen Daumenstempel (3c) zwecks Bedienung dieser Kolbenanordnung (3) im Zylinderabschnitt (2) aufweist, wobei an diesem Daumenstempel (3c) eine separate Aufnahmevorrichtung (4) montiert ist, die derart konfiguriert ist, einen Daumen eines Benutzers zwecks einhändigen Ansaugens während des Betriebs der Einweg-Injektionsvorrichtung (1) mit dieser Kolbenanordnung (3) wirkverbunden anzuordnen, **dadurch gekennzeichnet, dass** die separate Aufnahmevorrichtung (4) mittels einer -eine dauerhafte bzw. nicht wieder lösbare Verbindung ausbildende Rastelementanordnung (5)- an dem Daumenstempel (3c) montierbar ist und die Aufnahmevorrichtung (4) ferner eine Anzahl Führungselemente umfasst, die den Daumenstempel (3c) neben der Rastelementanordnung (5) zusätzlich einfasst.

2. Einweg-Injektionsvorrichtung (1) nach Anspruch 1, **dadurch gekennzeichnet, dass** die Aufnahmevorrichtung (4) einen Daumenring (6) und eine dem Daumenstempel (3c) zugewandte Auflagefläche (7) aufweist, die an mindestens zwei einander gegenüberliegenden Auflageenden (7a, 7b) Einfasswinkel (8a, 8b) umfasst, wobei an diesen Einfasswinkeln (8a, 8b) die Rastelementanordnung (5) den Daumenstempel (3c) einfassend ausgebildet ist, und die Einfasswinkel (8a, 8b) jeweils mindestens eine Öffnung (9a, 9b) aufweisen, durch die die als eine Anzahl Rastelemente (5a, 5b) ausgebildete Rastelementanordnung (5) bedienbar angeordnet ist, womit die Aufnahmevorrichtung (4) an dem Daumenstempel (3c) nicht wieder lösbar ausgestaltet ist.

3. Einweg-Injektionsvorrichtung (1) nach Anspruch 2, **dadurch gekennzeichnet, dass** mit den Rastelementen (5a, 5b) der Daumenstempel (3c) zwischen diesen Rastelementen (5a, 5b) und der Auflagefläche (7) der Aufnahmevorrichtung (4) unlösbar montierbar ist.

4. Einweg-Injektionsvorrichtung (1) nach einem der Ansprüche 1 bis 3, **dadurch gekennzeichnet, dass** die Aufnahmevorrichtung (4) mindestens eine Sollbruchstelle (10a, 10b, 10c) aufweist.

## Claims

1. A disposable injection device (1) comprising a cylinder portion (2) and a piston assembly (3) disposed therein, said piston assembly (3) having a first end (3a) closing said cylinder portion (2) and a second end (3b) having a thumb plunger (3c) for operating said piston assembly (3) in said cylinder portion (2), a separate receiving device (4) is mounted on said thumb plunger (3c) and is configured to operatively connect a user's thumb to said plunger assembly (3) for one-handed aspiration during operation of said disposable injection device (1), **characterized in that** said separate receiving device (4) is operatively connected to said plunger assembly (3) by means of a locking element arrangement (5) forming a permanent or non-releasable connection (6) to the thumb plunger (3c), and the receiving device (4) further comprises a number of guide elements which additionally enclose the thumb plunger (3c) in addition to the locking element arrangement (5).

2. Disposable injection device (1) according to claim 1, **characterized in that** the receiving device (4) has a thumb ring (6) and a bearing surface (7) facing the thumb plunger (3c), which bearing surface (7) comprises border angles (8a, 8b) at at least two opposite bearing ends (7a, 7b), wherein at these border angles (8a, 8b), the latching element arrangement (5) being designed to surround the thumb stamp (3c), and the surround angles (8a, 8b) each having at least one opening (9a, 9b) through which the latching element arrangement (5), which is designed as a number of latching elements (5a, 5b), is arranged such that it can be operated, with the result that the receiving device (4) is designed such that it cannot be released again on the thumb stamp (3c).

3. 3 Disposable injection device (1) according to claim 2, **characterized in that** the thumb plunger (3c) can be non-releasably mounted with the detent elements (5a, 5b) between these detent elements (5a, 5b) and the bearing surface (7) of the receiving device (4).

4. 4 Disposable injection device (1) according to any one of claims 1 to 3, **characterized in that** the receiving device (4) has at least one predetermined breaking point (10a, 10b, 10c).

## Revendications

1. Dispositif d'injection jetable (1) comprenant une section de cylindre (2) et un ensemble de piston (3) disposé à l'intérieur de celle-ci, lequel ensemble de piston (3) ferme la section de cylindre (2) à une première extrémité (3a) et présente à une deuxième extrémité (3b) un poinçon de pouce (3c) destiné à actionner cet ensemble de piston (3) dans la section de cylindre (2), un dispositif de réception séparé (4) étant monté sur le poinçon de pouce (3c), lequel est configuré de manière à disposer un pouce d'un utilisateur en liaison fonctionnelle avec cet agencement de piston (3) en vue d'une aspiration d'une seule main pendant le fonctionnement du dispositif d'injection à usage unique (1), **caractérisé en ce que** le dispositif de réception séparé (4) est monté au moyen d'un dispositif de fixation (5) qui forme une liaison permanente, c'est-à-dire une liaison de fixation d'éléments d'encliquetage (5) formant une liaison non amovible sur le poinçon du pouce (3c) et le dispositif de réception (4) comprend en outre un certain nombre d'éléments de guidage qui entourent le poinçon du pouce (3c) en plus du dispositif d'éléments d'encliquetage (5).

2. Dispositif d'injection à usage unique (1) selon la revendication 1, **caractérisé en ce que** le dispositif de réception (4) présente un anneau de pouce (6) et une surface d'appui (7) tournée vers le poinçon de pouce (3c), qui comprend des angles d'encadrement (8a, 8b) à au moins deux extrémités d'appui (7a, 7b) opposées l'une à l'autre, ces angles d'encadrement (8a, 8b), l'agencement d'éléments d'encliquetage (5) est réalisé de manière à entourer le poinçon du pouce (3c), et les angles d'encliquetage (8a, 8b) présentent chacun au moins une ouverture (9a, 9b) à travers laquelle l'agencement d'éléments d'encliquetage (5) réalisé sous la forme d'un certain nombre d'éléments d'encliquetage (5a, 5b) est disposé de manière à pouvoir être manoeuvré, le dispositif de réception (4) étant ainsi réalisé de manière à ne pas pouvoir être à nouveau détaché sur le poinçon du pouce (3c).

3. Dispositif d'injection jetable (1) selon la revendication 2, **caractérisé en ce que** les éléments d'encliquetage (5a, 5b) permettent de monter de manière inamovible le poinçon de pouce (3c) entre ces éléments d'encliquetage (5a, 5b) et la surface d'appui (7) du dispositif de réception (4).

4. Dispositif d'injection à usage unique (1) selon l'une des revendications 1 à 3, **caractérisé en ce que** le dispositif de réception (4) présente au moins un point destiné à la rupture (10a, 10b, 10c).
